# EUROPEAN PATENT SPECIFICATION

(11) **EP 4 014 164 B1**
(45) Date of publication and mention of the grant of the patent: **01.11.2023**
(21) Application number: 20758296.6
(22) Date of filing: 13.08.2020
(51) Int. Cl.: G06M 1/04, A61M 15/00

(54) **DOSE COUNTING MECHANISM**
DOSISZÄHLMECHANISMUS
MÉCANISME DE COMPTAGE DE DOSES

(30) Priority: 13.08.2019 GB 201911576
(43) Date of publication of application: 22.06.2022
(73) Proprietor: SANDOZ AG, 4056 Basel (CH)
(72) Inventor: COTTON, Matthew Richard, Cambridge CB4 0DS (GB); COCKER, Robin Craig, Cambridge CB4 0DS (GB); BLAIR, Arlo Christopher, Cambridge CB4 0DS (GB); CHRISTIE, Ewen Humphrey, Cambridge CB4 0DS (GB)
(74) Representative: Maiwald GmbH
(86) International application number: PCT/GB2020/051939
(87) International publication number: WO 2021/028693

(56) References cited:
- WO-A1-2004/001664
- WO-A1-2015/153624
- US-A1- 2013 139 815

## Description

The present invention relates to dose counting mechanisms for medicament inhaler devices, for example metered-dose inhaler (MDI) devices. In particular, the invention relates to an improvement of the indexing of a rotating wheel forming at least a part of the mechanical dose counting mechanism.

Accurate and reliable counting of doses is an important consideration for inhaler devices of all types, so that a user can be confident of the number of doses remaining for delivery. Counters can be broadly categorised into electronic dose counters and mechanical dose counters. Electronic counters are typically used where large numbers of doses need to be counted, but mechanical counters have the benefit of needing no power supply to register or display a dose count, so are generally preferred wherever possible.

Where a mechanical counter is required to count a large number of doses, multiple linked count wheels or drums are often employed to display the count. This avoids having to provide a physically large display surface on a single element of the counter, because each wheel need only display digits 0-9, and maybe end-of-life indicators/positions. A problem can arise, however, in ensuring that the first wheel, displaying the units value of the count, is sufficiently advanced/indexed during use of the device.

For a press-and-breathe pressurised metered-dose inhaler (pMDI) device, the indexing mechanism should be operated as part of the normal use-sequence. This generally means that the counter will be actuated by the movement of the medicament canister between its rest position and its fire point. This movement occurs over a relatively short distance, typically between 2mm and 3mm. For a counter where individual doses are registered by movement of a units wheel in a multi-wheel counter, the small movement must effect a relatively large rotation of 36° of the units wheel (i.e. a tenth of a rotation).

The timing, appearance, and accuracy of this indexing has potential implications for the user experience. Slowly indexing the counter throughout the act of depressing the canister can be slightly confusing to the user, especially if the counter is front-mounted and thus potentially visible during use. The partial indexing that may result can also be confusing, particularly if the counter remains in that state once the user has (even if just temporarily) stopped interacting with the device.

To avoid these problems, most dose counters only start to index at a defined commitment-point during the depression of the canister. There will also be a fire-point, i.e. a distance of canister depression at which the canister fires. Known inhaler devices are typically unable to tie these two points to the same depression distance unless some form of firing sensor is provided, but it is important that they are situated as close to each other as possible. However, care must be taken to ensure that, in all tolerance conditions, the counting commitment-point does not occur after the fire-point. After the commitment-point the dose counter will tend to complete an index, either during depression or on return to the rest position of the canister, regardless of whether the user continues depressing the canister or just releases it. Counting of a dose when there has been no delivery is undesirable, but delivery of a dose without indexing the counter should be minimised as far as is reasonably possible.

A counter indexing system will require energy-input, which can come directly from the user (in the form of increased canister-depression-force) and/or be harvested from unused force applied by the canister spring as the canister returns to its rest position. The lack of information surrounding the force-requirements for the canister spring may present reliability risks for the latter option, rendering it undesirable. However, care must be taken to ensure that the peak force experienced by the user during operation, if user force is directly harvested on the downward stroke, does not exceed a specified maximum force.

According to a first aspect of the invention there is provided an indexing mechanism as defined in the appended claim 1. Further optional features are recited in the associated dependent claims.

The mechanism comprises a counting component, a locking element for resisting movement of the counting component and a distinct driving element for driving movement of the counting component. The locking and driving elements have an initial position in which the locking element is engaged with the counting component, and the locking and driving elements are actuated by movement of a common element to move the locking element out of engagement with the counting component and to move the driving element, wherein the driving element applies a motive force to the counting component before the locking element fully disengages from the counting component. Engagement of the first element with the counting component may prevent movement of the counting component.

WO 2012/150427 discloses a dose counter mechanism where an actuator 2,102, 202 drives an index wheel 18,118,218 to advance a count wheel 26,126,226 once for every ten doses released. A hook 10,210 on the actuator drives the index wheel, and an engagement feature 17,217 on the actuator holds the count wheel in position between actuations. Both the hook and the engagement feature are moved simultaneously as the actuator is moved, so that engagement feature disengages from the count wheel as the hook moves towards engagement with the index wheel. The count wheel is free to advance when the hook engages and drives the index wheel.

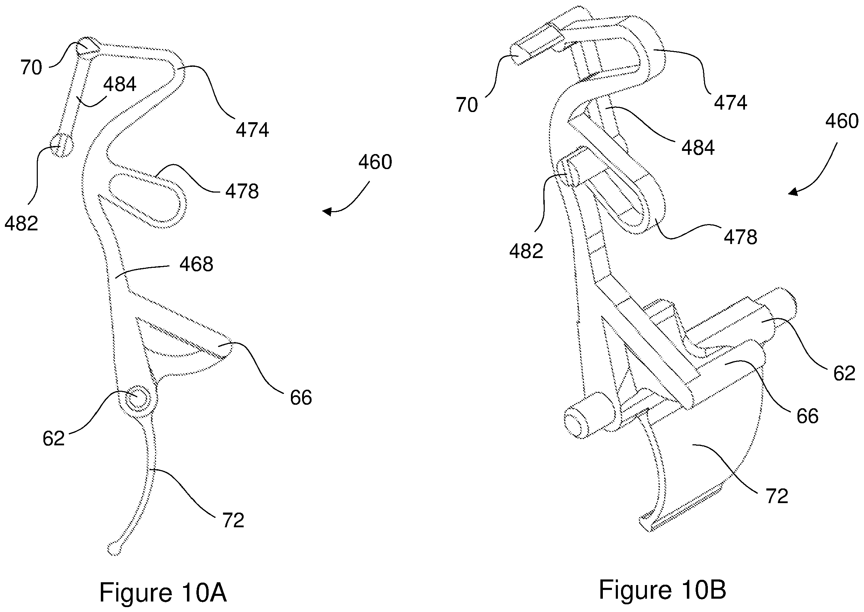

AU 2016204147 relates to a dose counter for a metered dose inhaler. As a canister is initially depressed, an actuator 240 moves anticlockwise to the position shown in Figure 10b. This moves a first pawl 242 into engagement with a wheel 230 as a second pawl 244 moves away. Continued depression of the canister causes rotation of the wheel 230 to the position shown in Figure 10c, which represents the end of a compression/firing stroke of the canister. On the return stroke, as shown in Figure 10d, the first pawl 242 moves clear of the wheel 230, and continued rotation of the wheel is then driven by engagement of the second pawl 244 during the rest of the rebound stroke until the mechanism reaches the position shown in Figure 10e. The two pawls are never simultaneously engaged with the wheel.

WO 2004/001664 relates to a dose counter incorporating three drums arranged on a common axis. A fixed pawl or resilient non-return leg 18 is provided for engagement with a ratchet wheel 71 to prevent back rotation of the counter drums. The non-return leg 18 is not moved out of engagement with the ratchet by an element of the driving mechanism. $

The driving element of the present invention may move into contact with the counting component from the first position to apply the motive force, or may be pre-engaged and apply a force in the first position prior to movement of the common element. Movement of the counting element may be driven by movement of the driving element from the first position.

The mechanism may incorporate some means of storing or charging energy behind or within the driving element. For example, the driving element may be provided on a resilient arm. The resilient arm may be deformed on/during engagement of the driving element with the counting component, or with a chassis or some other feature of a device housing the mechanism, such as an inhaler device, to store potential energy in the resilient arm.

The resilient arm may comprise a hinged or bent section. The hinged or bent section may be deformable or collapsible to adjust the spring rate of the resilient arm, or to provide a specific region with a different spring rate.

The resilient arm may comprise an abutment feature, for example a slot, loop, slot or similar, to resist deformation of the hinged or bent section in a first direction. This may provide a higher spring rate/resistance to deformation in said first direction than in one or more other directions.

The driving component may be out of engagement with the counting component in the initial position, and actuation of the driving element by the common element may then move the driving element into engagement with the counting component to apply a motive force to the counting component.

The driving element may engage with a slot in the counting component.

The slot in the counting component may comprise a cam surface for engagement with the driving element. The slot in the counting component may comprise first and second cam surfaces for engagement with the driving element. The first and second cam surfaces may be engaged in opposite movement directions of the of the driving element, so as to deform the resilient arm in opposite directions during use. Different spring rates/resistances to deformation may be provided, for example by an abutment feature as previously described.

The indexing mechanism may further comprise a restoring element providing a biasing force to urge the locking and/or driving element back to the initial position. Providing different spring rates as discussed may reduce the biasing force needed to urge the locking and/or driving element back to the initial position. The restoring element may comprise a spring, for example a metal or polymer spring.

The spring may be a leaf spring and may be part of an integrally formed component which also comprises the locking and/or driving element.

The locking element may engage with a recess in the counting component.

The locking and driving elements may be provided on separate components such that the indexing mechanism may comprise an indexing actuator and a locking actuator, or the locking and driving elements may provided on a common component such that the mechanism comprises a single indexer component.

The counting component may be a rotatable component and movement of the counting element may comprise rotation. The rotatable component may be a count wheel of a dose counter.

Movement of the common element may comprise depression of the common element, for example by a user. The common element may be an MDI canister.

The invention also provides a method of indexing a counter as defined in the appended claim 11.

Further optional features are recited in the associated dependent claims.

The method comprises sequential steps of:
A. engaging a locking element with a counting component to prevent movement of the counting component; and subsequently
B. moving a driving element to apply a motive force the counting component; and
C. moving the locking element out of engagement with the counting component.

The locking and driving elements are moved in steps B and C by movement of a common element, the driving element applies the motive force to the counting component before the locking element fully moves out of engagement with the counting component and the driving element drives movement of the counting component once the locking element is fully out of engagement with the counting component.

The locking and driving elements may be moved simultaneously in steps B and C.

Step B may comprise storing energy to drive movement of the counting component.

The counting component may be rotatable and the driving element may drive rotation of the counting component. The driving element may further hold the counting component in a rotated position after driving rotation.

The method may comprise the further step of:
D. moving the locking element back into engagement with the counting component. The locking element may be moved in step D by a spring biasing force.

Movement of the locking and/or driving element may comprise rotation around a pivot.

Movement of the common element may comprise depression of the common element.

The method may be implemented using an indexing mechanism as previously described.

Practicable embodiments of the invention are described in further detail below with reference to the accompanying drawings, of which:
Figure 1 shows an exploded view of a pMDI including a dose counter comprising an indexing mechanism according to the present invention;
Figure 2 shows a perspective view of a counter wheel from the dose counter of Figure 1;
Figure 3 shows a perspective view of a locking actuator from the indexing mechanism of Figure 1;
Figure 4 shows a perspective view of an indexing actuator from the indexing mechanism of Figure 1;
Figure 5 shows a front view of the assembled indexing mechanism of Figure 1;
Figures 6A to 6F are front views of the indexing mechanism during operation;
Figure 7 shows a perspective view of an alternative indexing actuator for the indexing mechanism of Figure 1;
Figure 8 shows a perspective view of a further alternative indexing actuator for the indexing mechanism of Figure 1;
Figures 9A and 9B show a further alternative indexing actuator for the indexing mechanism of Figure 1;
Figures 10A and 10B show a further alternative indexing actuator for the indexing mechanism of Figure 1;
Figures 11A and 11B show a further alternative indexing actuator for the indexing mechanism of Figure 1;
Figure 12 shows an exploded view of an alternative indexing mechanism according to the present invention; and
Figure 13 shows a front view of the alternative indexing mechanism of Figure 12.

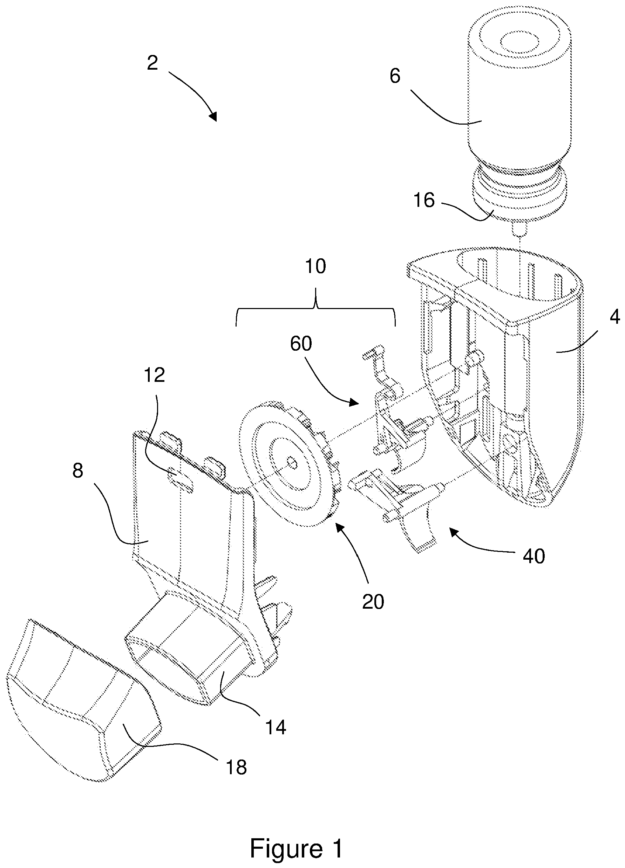

An exploded view of a press-and-breathe pressurised metered-dose inhaler (pMDI) device 2 is shown in Figure 1. The pMDI 2 comprises a rear housing/body 4, for receiving a canister 6 of medicament with a valve stem at a lower end 16 as shown, and a front housing 8. Components of a dose counter 10 are shown between the front and rear housings 4,6. The individual components include a counter wheel 20, a locking actuator 40 and an indexing actuator 60, all of which will be described more fully later.

The front housing 8 further comprises a window 12 through which a portion of the counter wheel 20 can be viewed, and a mouthpiece 14 providing fluid connection to the medicament canister 6 when the pMDI is assembled. The precise internal geometry of the rear housing 4 to provide the fluid communication and facilitate release of a dose from the canister 6 is unimportant to the present invention, and various suitable configurations will be familiar to a skilled reader. A mouthpiece cover 18 for closing and protecting the mouthpiece 14 is also provided, as is conventional.

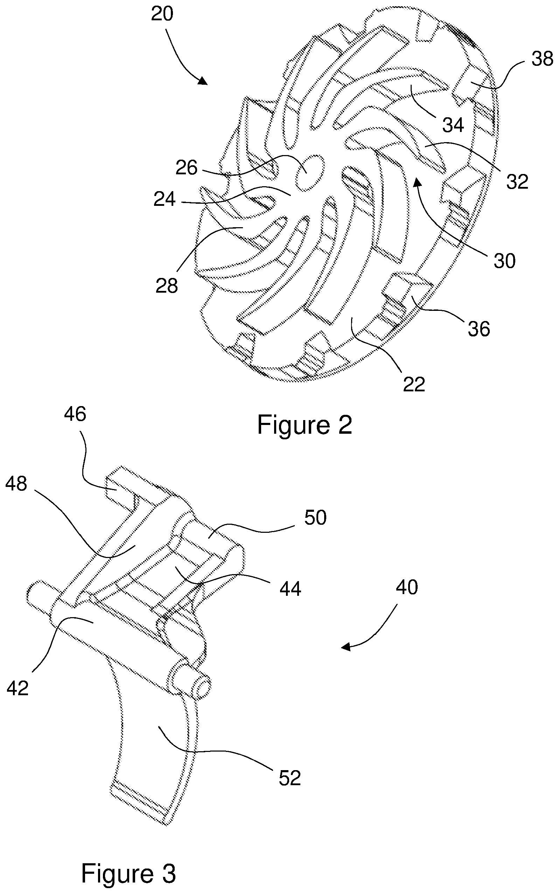

A rear perspective view of the counter wheel 20 is shown in Figure 2. The counter wheel 20 comprises a generally flat disc portion 22 and a charging feature 24 raised from the rear surface of the disc portion 22. A centrally positioned mounting hole 26 is provided for mounting the counter wheel 20 on a peg provided in the rear housing 4.

The charging feature 24 comprises ten evenly spaced curved fingers 28 defining ten slots 30 therebetween. Each curved finger 28 provides a first camming surface 32 and a second camming surface 34, with each slot 30 provided between the first camming surface 32 of one curved finger 28, and the second camming surface 34 of an adjacent curved finger 28. The curved fingers 28 are shaped so that each slot 30 is wider at an end that is nearest the outer circumference of the counter wheel 20.

The outer circumference of the disc portion 22 of the counter wheel 20 is provided with ten evenly spaced locking recesses 36, each of which comprises one chamfered edge 38, angled to the radius of the counter wheel 20.

The various features on the rear of the counter wheel 20, specifically the first and second camming surfaces 32,34 and locking recesses, cooperate with parts of the locking actuator 40 and indexing actuator 60 to provide an indexing system in accordance with the present invention.

As shown in Figure 3, the locking actuator 40 comprises a central mounting pin 42 to be received in recesses in the front and rear housings 4,8 to provide a pivoting connection. A substantially rigid arm 44 extends from the mounting pin 42, and a locking peg 46 is supported on an elevated part 48 of the substantially rigid arm 44. The locking peg 46 extends laterally from the elevated part 48 of the substantially rigid arm 44, generally parallel to the mounting pin 42. Also provided on the substantially rigid arm 44, but spaced from the locking peg 46 is a bearing surface 50. A spring arm 52 also extends from the mounting pin 42, generally perpendicular to the substantially rigid arm 44.

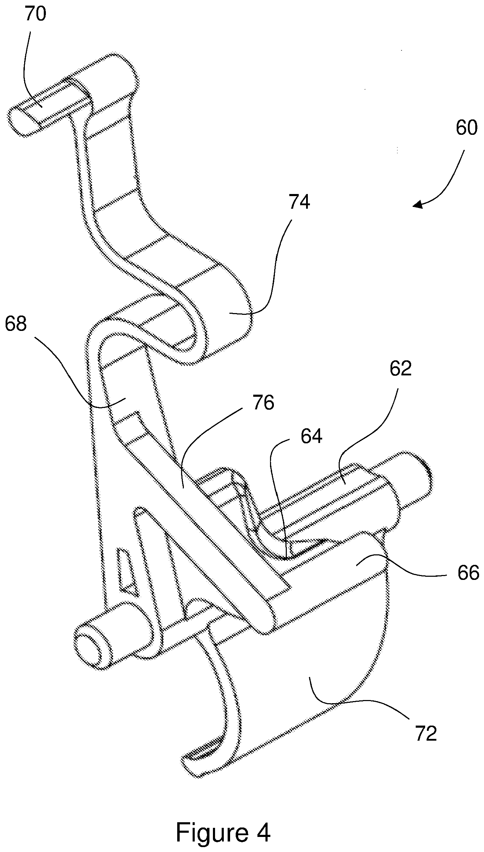

The indexing actuator 60 is shown in Figure 4. Like the locking actuator 40, the indexing actuator 60 comprises a central mounting pin 62 to provide a pivoting connection in the front and rear housings 4,8, and a substantially rigid arm 64 extends from the mounting pin 62 and provides a bearing surface 66. The indexing actuator 60 also provides an indexing arm 68, generally perpendicular to the mounting pin 62, with an indexing peg 70 provided at its upper end remote from the mounting pin 62. A spring arm 72 is provided as in the locking actuator 44 previously described.

The indexing arm 68 comprises a hairpin bend 74, hereafter referred to as a hairpin 74, part way along its length, and is thicker at a lower end, adjacent the mounting pin 62, than at the upper end, adjacent the indexing peg 70. A diagonal brace 76 is also provided between the substantially rigid arm 66 and the indexing arm 68, at a position between the mounting pin 62 and the hairpin 74. A first part of the indexing arm 68 therefore has a relatively high rigidity, and a second part is more easily deformable.

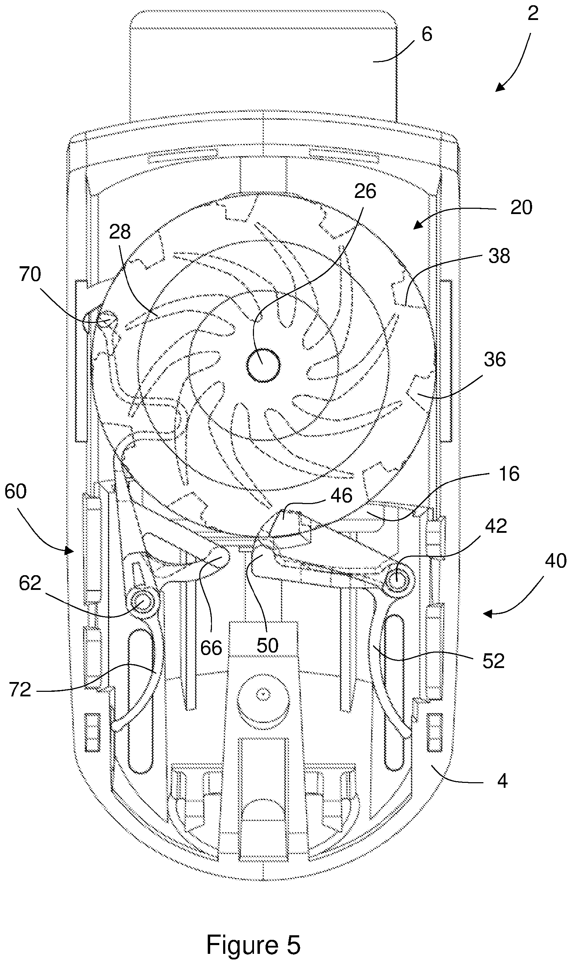

Figure 5 shows the assembly of the counter wheel 20, locking actuator 40 and indexing actuator 60 within the rear casing 4 of the inhaler 2 to provide a complete dose counter mechanism. In the described embodiment, each of the counter wheel 20 locking actuator 40 and indexing actuator 60 is moulded as a single piece component in a plastics material.

Each of the locking actuator 40 and indexing actuator 60 is pivotally mounted to the rear housing 4 at its respective mounting pin 42,62, and the free ends of the spring arms 57,72 are bearing against an inner surface of the rear housing 4. The counter wheel 20 is mounted via its central mounting hole 26 so at to be rotatable relative to the rear housing 4.

The lower end 16 of a canister 6 can be seen just above the bearing surfaces 50,66 of the locking actuator 40 and indexing actuator 60. In this position, the indexing peg 70 of the indexing actuator 60 is adjacent but spaced from the end on one of the curved fingers 28, and the locking peg 46 of the locking actuator 40 is engaged with one of the locking recesses 36 provided around the edge of the counter wheel 20. The chamfered edge 38 at one side of each of the locking recesses 36 can also be seen clearly in Figure 5.

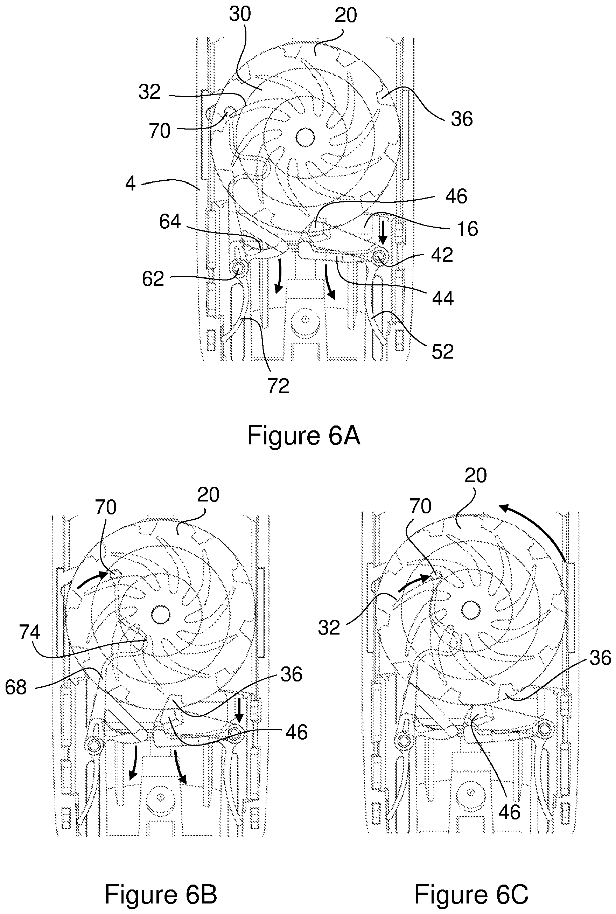

Operation of the dose counter will now be described with reference to Figures 6A to 6F. The locking actuator 40 and indexing actuator 60 are both rotated from their rest positions during use by movement of the canister 6, and both are rotated back to their rest position after indexing by their respective spring arms 52,72, which are anchored on the caseworks/rear housing 4 of the inhaler device 2.

In Figure 6A, the cannister 6 has been depressed a small amount so that its lower end 16 has moved vertically downwards to bear on the bearing surfaces 50,66 of the locking actuator and indexing actuator 40,60. This rotates the substantially rigid arm 64 of the indexing actuator 60 clockwise around its mounting pin 62 and rotates the substantially rigid arm 44 of the locking actuator 40 anticlockwise around its mounting pin 42. Rotation of the substantially rigid arm 64 of the indexing actuator 60 causes corresponding rotation of the indexing arm 68 so that the indexing peg 70 engages with the first camming surface 32 of a slot 30 of the counter wheel 20. The simultaneous rotation of the substantially rigid arm 44 of the locking actuator 40 causes the locking peg 46 to start moving out of the locking recess 36. The spring arms 52,72 are prevented from rotating by the walls of the rear housing 4, so they resiliently deform to provide a restoring force to the locking actuator and indexing actuator 40,60.

Further downward movement of the lower end 16 of the canister 6 further rotates the indexing actuator 60, causing the indexing peg 70 to move along the first camming surface 32 towards the centre of the counter wheel 20. The locking actuator 40 also continues to rotate, but the locking peg 46 remains in engagement with a locking recess 36. Accordingly, the counter wheel 20 is prevented from rotation, and the indexing arm 68 resiliently deforms as the indexing peg 70 moves along the first camming surface 32. As shown in Figure 6B the locking peg 46 is just moving clear of a locking recess 36, and the hairpin 74 has opened out as part of the deformation of the indexing arm 68.

The length of the indexing arm 68 locates the drive peg 70 relatively far from the mounting pin 62 of the of the indexing actuator 60, which serves to amplify movement of the drive peg 70 during operation.

Figure 6C shows the dose counter part-way through an indexing operation. The locking peg 46 has moved clear of the locking recess 36 to an unlocked position so that the counter wheel 20 is free to rotate. Energy stored by the resilient deformation of the indexing arm 68 can then drive the anticlockwise rotation of the counter wheel 20, moving the indexing peg 70 further along the first camming surface 32. The amplified movement of the indexing peg 70 helps to ensure that sufficient elastic potential energy is stored in the indexing arm 68 to power an index and advance the counter wheel 20 the required distance.

The point at which the locking peg 46 clears the locking recess 36 to unlock the counter wheel 20 represents a commitment point before which depression of the canister 6 will not result in a dose being counted. The location of the locking peg 46 relatively close to the mounting pin 42 of the locking actuator 40 helps to minimise variation of this commitment point due to manufacturing tolerances.

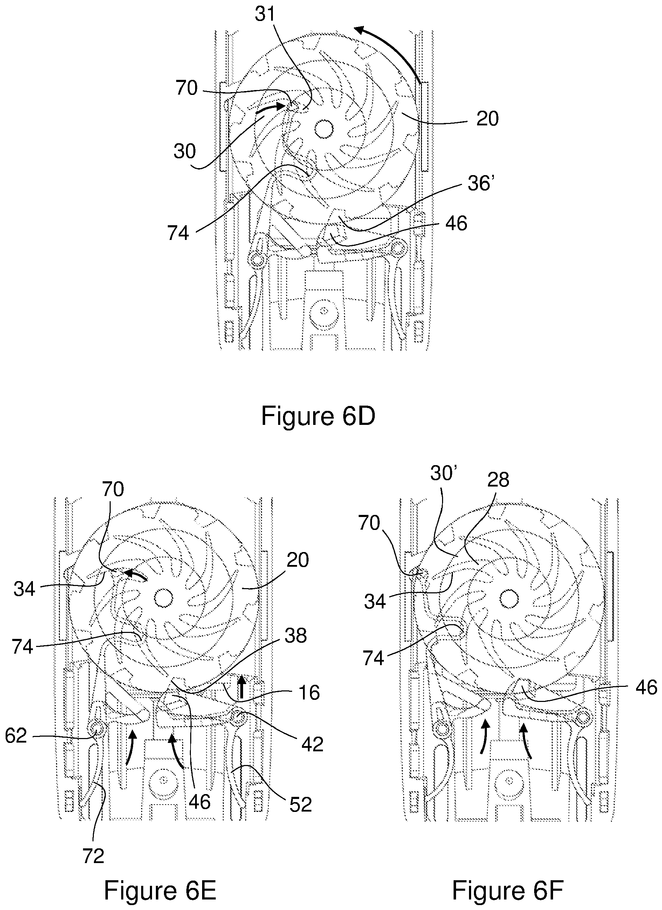

The rotation of the counter wheel 20 continues until, as shown in Figure 6D, the hairpin 74 returns to its undeformed position. The indexing peg 70 moves into an end portion 31 of the slot 30 which is angled relative to the remainder so that further movement of the indexing peg 70 towards the centre of the counter wheel 20 imparts no rotational drive to the counter wheel 20, and indeed maintains the rotational position of the counter wheel 20. This provides some dwell time at the end of an indexing operation. The locking peg 46, at this point, is aligned with the next locking recess 36' around the circumference of the counter wheel 20.

When pressure on the canister 6 is released, its lower end 16 begins to move vertically upwards clear of the bearing surfaces 50,66 of the locking actuator 40 and indexing actuator 60, as shown in Figure 6E. The energy stored by resilient deformation of the spring arms 52,72 causes clockwise rotation of the locking actuator 40 and anticlockwise rotation of the indexing actuator 60 around their mounting pins 42,62. The clockwise rotation of the locking actuator 40 causes the locking peg 46 to engage with the chamfered edge 48 of the next locking recess 36', and then move into a locked position in the next locking recess 36' to again resist rotation of the counter wheel 20. The chamfered edge 38 provides a lead-in feature to help account for any slight misalignment of the counter wheel 20. The anticlockwise rotation of the indexing actuator 60 causes the indexing peg 70 to move radially outwards along the slot 30. The indexing peg 70 engages with the second camming surface 34 of the slot 30 during this movement, and this causes a compression in the hairpin 74 of the indexing arm.

Figure 6F shows the end of an indexing operation, with the mechanism essentially returned to its rest configuration as shown in Figure 5, but advanced by one dose. The continued rotation of the locking actuator 40 and indexing actuator 60 has fully engaged the locking peg 46 with the recess 36' in the counter wheel 20 and moved the indexing peg 70 clear of the end of the second camming surface 24. The energy stored during compression of the hairpin 74 causes the indexing peg 70 to spring up past the end of the second camming surface 24 ready to engage with the next slot 30' of the counter wheel 20. Compressing the indexing arm 68 also counteracts the effects of applying tensile stress during indexing, ensuring that any creep effects are minimised.

It will be appreciated that the operation described in Figures 6A-6F corresponds to one full depression and release of the canister 6, i.e. the delivery of a single dose of medicament. Since the counter wheel 20 provides ten slots 30 and ten locking recesses 36, ten actuations will result in one full rotation of the counter wheel 20.

It will be understood that the indexing arm 68 can be resiliently deformed and stressed during operation by the camming surface 32,34 via compression or extension of the hairpin 74 or simply by bending in the arm 68. Although primarily designed to rely on deformation at the hairpin 74, it is likely that some bending of the arm 68 will also occur. Indeed, it is possible to design an alternative indexing arm that will store elastic potential energy by deforming more equally in both of these modes simultaneously.

The applicant has also investigated the possibility of using different deformation modes and/or directions to achieve differential spring forces in an indexing arm during its two stages of being stressed. This would allow, for example, a higher spring force to be provided when an indexing arm is deformed during engagement with the first camming surface 32, and a lower spring force to be provided by the same arm during engagement with the second camming surface 34. The energy input for the indexing of the counter wheel 20 needs to be relatively high, to ensure that the indexing operation is reliably completed. In contrast, the spring force during reset of the arm should ideally be as low as possible, since it directly impacts the size and strength of the spring arm required to return the indexing arm under all environmental and/or tolerance conditions, and thus the force that the user must put into depressing the canister 6 and stressing the spring arm during use.

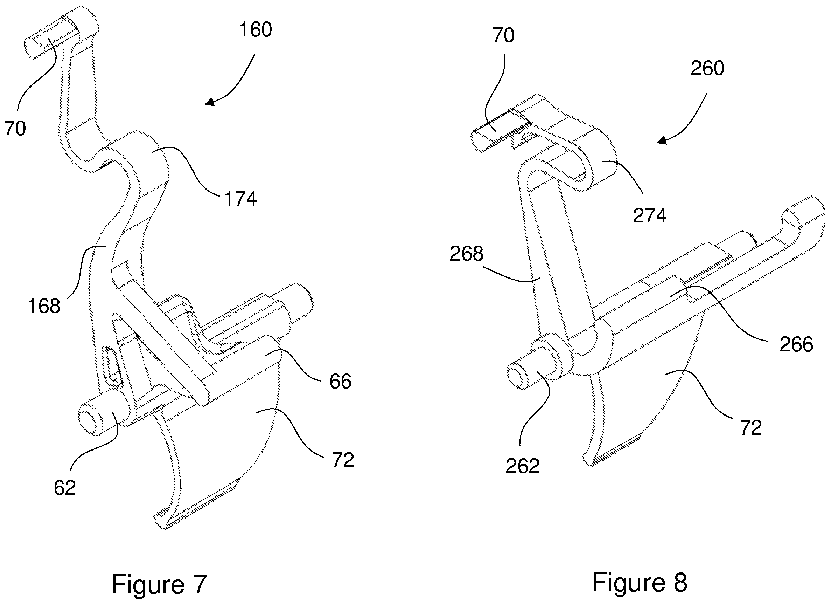

Some possible alternative indexing actuators for the present invention are illustrated in Figures 7 to 11B.

Figures 7 and 8 show indexing actuators 160,260 that are broadly similar to the indexing actuator described above. The indexing actuator 160 of Figure 7 comprises an alternative hairpin 174 in the indexing arm 168. Essentially, the hairpin 174 of this first alternative indexing actuator 160 is rotated relative to that shown in Figure 4. The remaining components of the indexing actuator 160, such as the indexing peg 70, mounting pin 62, spring arm 72 and bearing surface 66 are as previously described.

Figure 8 shows a second alternative indexing actuator 260, which illustrates how the dimensions and design of an indexing actuator may be modified according to different packaging constraints. A shorter indexing arm 268 is provided, with the hairpin 274 located at the upper end of the indexing arm 268 immediately adjacent the indexing peg 70. The mounting pin 262 and bearing surface 266 are longer than those in the previously described indexing actuators 60,160. A spring arm 72 is again included to provide a restoring force as before.

This indexing actuators 160,260 of Figures 7 and 8 do not feature any clear differentiation between the index-energy spring rate (during depression of the canister 6) and the reset spring rate. Both modes of use will cause a similar mixture of bending along the length of the indexing arm 168,268 and extension or retraction of the length of the arm 168,268 via expansion or contraction of the hairpin feature 174,274.

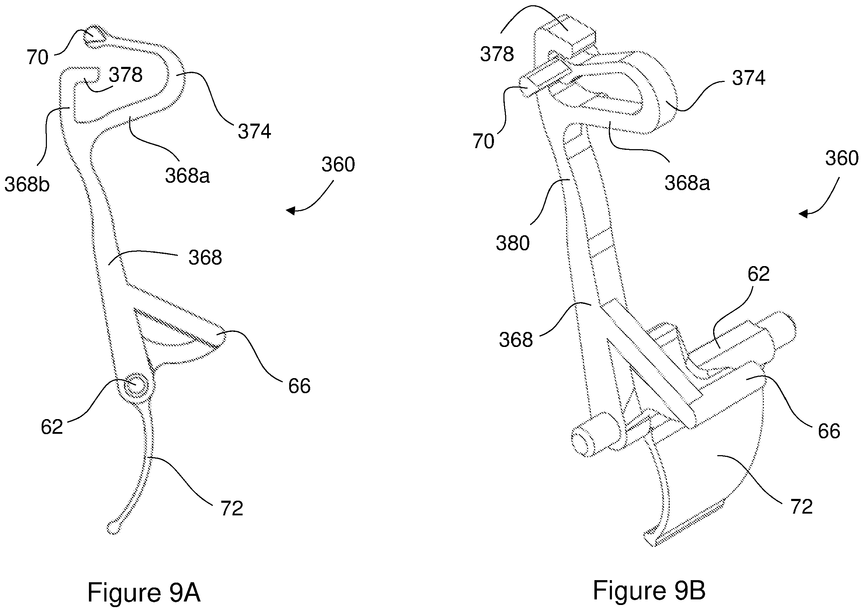

Figure 9A shows a front view of a further alternative indexing actuator 360, which has a slightly more complex design to provide different spring forces in the two modes of use outlined above. The side view of Figure 9A shows the indexing actuator 360 as moulded. The design of the lower part of the indexing actuator 360, including the mounting pin 62, bearing surface 66 and spring arm 72, is essentially the same as that of the indexing actuators 60,160 shown in Figures 4 and 7. The indexing arm 368 has a more complex design, with two branches 368a,368b at an upper end. The indexing peg 70 is provided at the end of the first branch 368a, which incorporates a hairpin 374 similar to the hairpin 74 of the first indexing actuator 60 of Figure 4. The second branch 368b includes a lip/end-stop 378 at its free end.

A perspective view of the same indexing actuator 360 in its primed condition is shown in the perspective view of Figure 9B. The end of the first branch 368a, with the end of the indexing arm 368 comprising the indexing peg 70, has been hooked underneath the end-stop 378 at the end of the second branch 368b. The indexing peg 70 can still move downwards, or towards the mounting pin 62, with a relatively low force by contracting the hairpin 374. However, the end-stop 378 prevents the hairpin 374 from opening/expanding from the position shown in Figure 9B so that movement of the indexing peg 70 relative to the arm 368 away from the mounting pin 62 is resisted. As a result of this design, depression of a canister 6 results in energy storage solely through bending of the indexing arm 368 as the indexing actuator 360 is rotated during the indexing operation. This provides a relatively high spring force, which can be reduced if required by including a thinner region 380 in the indexing arm 368, to ensure there is sufficient energy to index the counter. During a return or reset stroke, in the opposite direction, the hairpin 374 can also collapse to providing a lower overall spring force, meaning that the size and strength of the spring arm 72 can be minimised.

Figures 10A and 10B show similar views of a fourth alternative indexing actuator 460, which achieves a similar outcome via a different design. Again, Figure 10A shows the indexing actuator 460 as moulded, and Figure 10B in its primed condition. In the fourth alternative indexing actuator 460 a loop 478 is provided part way up the indexing arm 468, and a stop peg 482 is provided on the end of an extension potion 484 extending from the indexing peg 70 at the end of the indexing arm 468.

In the primed condition the stop peg 482 is engaged with the loop 478 by bending the hairpin 474. The engagement of the stop peg 482 with the loop 478 essentially performs a similar function to the end-stop 378 in the third alternative indexing actuator 360, providing a cam track to limit the movement of the indexing peg 70. During depression of the canister 6 the indexing peg 70 cannot move away from the mounting pin 62 through expanding the hairpin 474, because this movement is prevented by the stop peg 482 and loop 478. Movement during the indexing can therefore only be achieved through bending of the indexing arm 468 as the indexing actuator 460 rotates. In contrast, the stop peg 482 can move within the loop 478, towards the mounting pin 62, during reset. The hairpin 474 is therefore free to compress in this mode of operation, which provides a relatively lower spring force. As above, this provides a desirable balance of a larger spring force during indexing, and a lower spring force for reset.

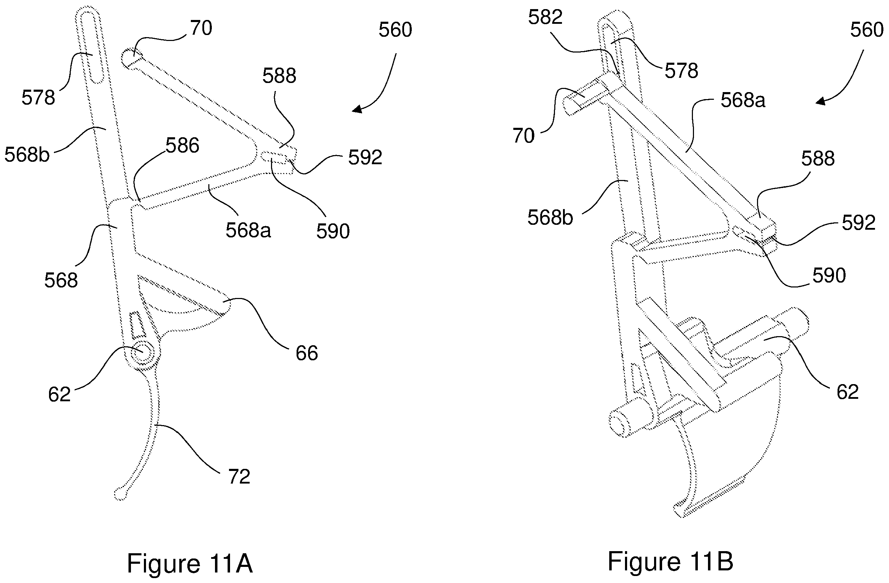

A final example showing a fifth alternative indexing actuator 560 in moulded and primed configurations is provided with reference to Figures 11A and 11B. In this embodiment, the indexing arm 568 can again be considered to split into first and second branches 568a,568b at an upper end, with the indexing peg 70 provided at the end of the first branch 568a. A locating peg 582 projects from an opposite side of the first branch 568a aligned with the indexing peg 70, and is received in a camslot 578 provided on the second branch 568b to limit the movement of the indexing peg 70 to a single radial line to or from the mounting pin 62 of the indexing actuator 560. The first branch 568a also comprises first and second hinges 586,588. The first hinge 586 is provided at a lower end of the first branch 568a, and the second hinge 588 is provided between two halves of the first branch 568a so that the first branch forms a 'V' shape. The second hinge 588 includes an open section 590 towards its outer side, with the apex of the 'V' being provided by two abutting end-stops 592.

In use, radial movement of the indexing peg 70 away from the mounting pin 62, while storing up energy for indexing, quickly causes the end-stops 592 in the second hinge 588 to engage and `lock out' the hinge 588 preventing further opening/expansion. Further movement of the pin is possible only by bending the two limbs of the first branch 568a to open out the 'V'. This results in a relatively high spring force, sufficient for the indexing operation.

Radial movement of the indexing pin 70 towards the mounting pin 62, during reset, can be achieved with a relatively lower force, because the end-stops 592 move apart allowing the two hinges 586,588 to collapse the 'V' with minimal bending of either limb of the first branch 568a so that the indexing pin 70 can move down the cam slot 578 under minimal resistance. The necessary restoring force to be provided by the spring arm 72 is therefore minimised.

It will be understood that the movement of the indexing pin 70 in the fifth alternative indexing actuator 560 regulated by the first branch 568a of the indexing arm 568 and the cam slot 578. The remainder of the indexing arm 568, including the second branch 568b, can be made relatively stiff to minimise bending in the arm 568, so that the behaviour of the indexing actuator 560 under load, and in particular the precise position of the indexing pin 70, is as predictable as possible.

All of the indexing actuators 60,160,260,360,460,560 described above are intended to be used with a locking actuator 40 substantially as shown and described in Figure 3.

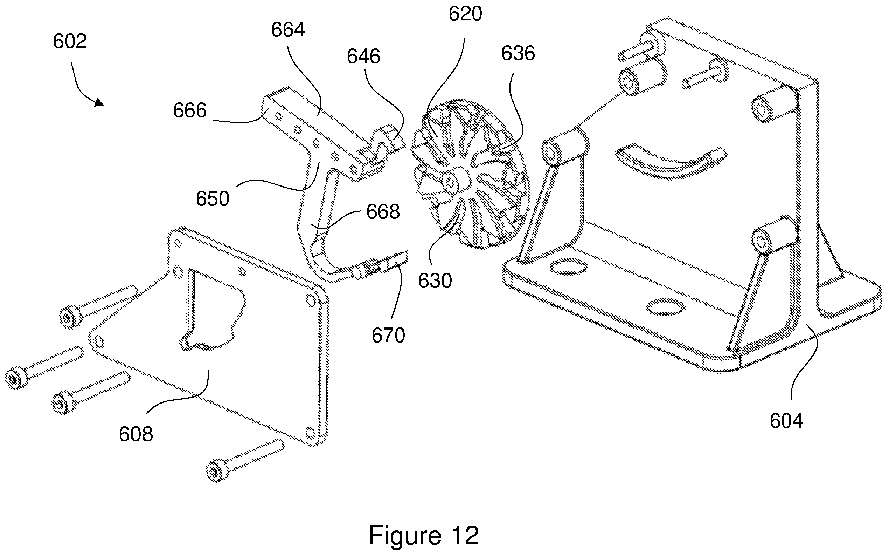

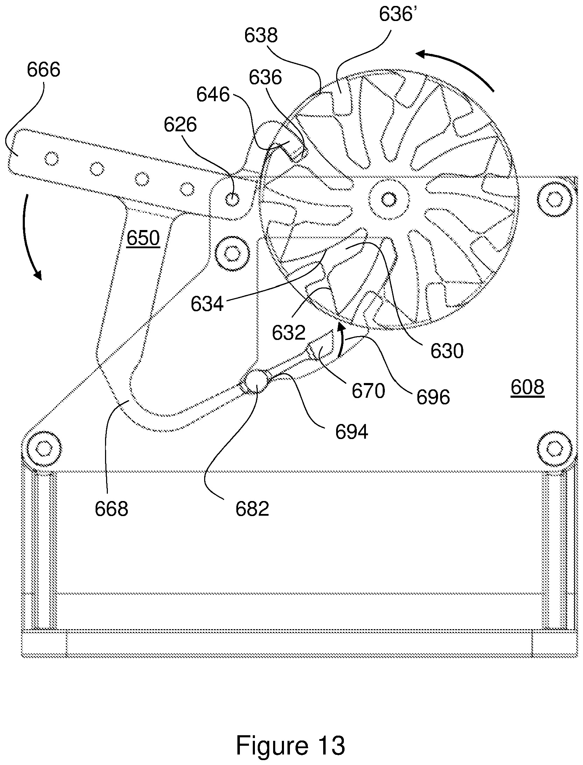

An alternative embodiment 602 of the invention is shown in an exploded perspective view in Figure 12, and an assembled side view in Figure 13. This alternative embodiment 602 combines the indexing actuator 60 and locking actuator 40 of the invention into a single indexer component 650. As shown, the alternative embodiment 602 is in 'rig' form for experimental/development purposes. The counter wheel 620 and indexer 650 are mounted to a stand 604 and secured in place by a front plate 608, and the indexer 650 includes a lever 666 to be operated either by hand or by force-testing equipment, rather than specific features for interacting with a pMDI canister. It will be understood, however, that the arrangement could readily be modified for use in a pMDI device by a skilled reader.

In a similar manner to the counter wheel 20 of Figure 2, the counter wheel 620 of this alternative embodiment comprises ten evenly spaced curved slots 630 and ten evenly spaced locking recesses 636. The indexer 650 is broadly 'J' shaped, having a generally horizontal top section 664 providing the lever 666 and a curved indexing arm 668 with a flexible end section. The top section 664 comprises a locking hook 646 at the end opposite the lever 666, and an indexing tooth 670 is provided at the end of the curved indexing arm 668.

As shown in the assembled view of Figure 13, the locking hook 646 is engaged with one of the locking recesses 636 in the counter wheel 620, and the indexing tooth 670 is positioned below the counter wheel 620. The mounting pivot 626 of the indexer 650 is located adjacent the locking hook 646 and is spaced by a greater distance from the indexing tooth 670 so that rotation of the indexer 650 will cause a greater movement of the indexing tooth 670 than the locking hook 646.

In use, a downward pressure is applied to the lever 666 to cause anticlockwise rotation of the indexer 650. The anticlockwise rotation causes a locating peg 682 on the indexing arm 668 to move over a small camming surface 694 on the front plate 608, flexing the end of the indexing arm 668 towards the pivot 626 in the direction indicated by arrow 696. Continued rotation of the indexer 650 brings the indexing tooth 570 into contact with an angled surface of one of the slots 630 which acts as a first camming surface 632 on the counter wheel 620. The locking hook 646 remains engaged with a locking recess 636 at this stage, so that continued anticlockwise rotation of the indexer 650 results in the indexing arm 668 flexing further in the direction of arrow 696.

When the lever 666 has been depressed sufficiently to rotate the locking hook 646 clear of the locking recess 636 in the counter wheel, the spring force stored in the indexing arm 668 will then be transferred to the counter wheel 620 via the indexing tooth 670, rotating the counter wheel 620 anticlockwise. The disengagement of the locking hook 646 defines a commitment point that would, in practice, be coordinated with the release of a dose from a pMDI canister 6.

Anticlockwise rotation of the counter wheel 620 is limited by engagement of the indexing tooth 670 with a second camming surface 634 on the opposite side of the slot 630 from the first camming surface 632. Continued downward pressure on the lever 666, indicative of further downward movement of a pMDI canister 6, results in continued rotation of the indexer 650 and moves the indexing tooth within the slot 630. This maintains a rotational position of the counter wheel 620 and provides some dwell time at the end of an indexing operation.

During subsequent rotation of the indexer 650 in a clockwise direction, the engagement of the indexing tooth 670 in the slot 630 initially maintains the rotational position of the counter wheel 620 until the locking hook 646 engages with the next locking recess 636'. A chamfered edge 638 is provided so that the engagement can to help account for any slight misalignment of the counter wheel 620if necessary.

With the counter wheel 620 again locked against rotation, continued movement of the indexing tooth 670 along the second camming surface 634 as the indexer rotates clockwise serves to flex the indexing arm 668 in the opposite direction to arrow 696. This stores energy in the indexing arm 668 so that is springs past the end of the slot 630 once disengaged, ready for a subsequent indexing operation. A coil spring may be provided at the pivot 626 to store energy during the anticlockwise rotation and drive the clockwise rotation of the indexer 650. In a fully realised system, other resilient/spring biasing means, such as a torsion spring or integrated leaf spring(s), may be provided to provide a restorative force to the indexer 650.

It should be understood that the embodiments described above comprise a single counter wheel for simplicity. The principles described would be scalable for a larger number of doses by using larger wheels with a greater number of slots 30,630 and locking recesses 46,646, or by employing multiple linked count wheels with the described counter wheel 20,620 providing the unit count.

As noted above, the distance of translation of the canister during depression is relatively small compared to the required movement of the dose-counter wheel. Therefore, it may be beneficial for the distance of movement of the feature that interacts with the counter-wheel, and enables the component to store the required energy for rotation of the counter wheel, to be 'geared up' or otherwise amplified. This will help to provide enough movement to effect the necessary index, but may also increase the tolerances on the position of that feature at any given moment.

To counteract the poor positional tolerances on this indexing feature, the locking feature of the mechanism - which sets the trigger-point for the indexing of the counter wheel relative to the position of the canister - can be kept as a separate feature, and situated close to its axis of rotation (or other point joining it to the chassis) to ensure good tolerances and good positional control of the trigger-point.

Once the trigger-point has been reached, and the indexing feature has indexed the counter wheel, there are a variety of possible ways in which the counter wheel can be stopped in the desired angular position:
- The indexing feature can be allowed to stop in its natural, unstressed position;
- To improve the angular positioning of the previous option, the path of the indexing feature can be planned in such a way that its final movements are substantially radial relative to the counter wheel, and/or interact with a cam profile such that said final movements do not induce rotation in the counter wheel, meaning that loose tolerances on the final position of the indexing feature can be translated to tight tolerances on the angular position of the counter wheel;
- The indexing feature, or another feature of the component on which it is borne, may be designed to hit an end-stop that positions it - and thus the counter wheel - more closely than relying on the final position of the loosely-toleranced indexing feature;
- The locking feature may function as a form of escapement, both releasing and catching the counter wheel to provide a tightly-toleranced end-stop to its rotation; this would probably involve two instances of indexing movement per complete index of the counter wheel.

Various other modifications would also be apparent to a skilled reader. As such, it is emphasised that the forgoing description is provided by way of example only, and is not intended to limit the scope of protection as defined with reference to the appended claims.

Examples of alternative configurations include reversing the direction of rotation of the counter wheel, reversing the direction of radial movement of the indexing peg/tooth (i.e. from towards the centre of the counter wheel toward its outer edge) and/or providing a similar dual charge/release system in a non cam-driven system, for example using a more direct, tangential driving of the counter wheel.

## Claims

1. An indexing mechanism for a counter (10), comprising a counting component (20,620), a locking element (46,646) for resisting movement of the counting component and a distinct driving element (70,670) for driving movement of the counting component, the locking and driving elements having an initial position in which the locking element (46,646) is engaged with the counting component (20,620), **characterized in that**
the locking and driving elements being actuated by movement of a common element (16,666) to move the locking element (46,646) out of engagement with the counting component (20, 620) and to move the driving element (70,670), wherein the driving element (70, 670) applies a motive force to the counting component (20,620) before the locking element (46,646) fully disengages from the counting component (20,620).

2. An indexing mechanism according to claim 1, wherein engagement of the locking element (46,646) with the counting component (20,620) prevents movement of the counting component (20,620).

3. An indexing mechanism according to claim 1 or 2, wherein the driving element (70,670) is provided on a resilient arm (68,368,468,668) which is preferably deformed on engagement of the driving element (70,670) with the counting component (20,620) to store potential energy in the resilient arm (68,368,468,668).

4. An indexing mechanism according to claim 3, wherein the resilient arm (68,368,468,668) comprises a hinged or bent section (74,374,474).

5. An indexing mechanism according to claim 4, wherein the resilient arm (68,368,468,668) comprises an abutment feature (378,478) to resist deformation of the hinged or bent section (374,474).in a first direction.

6. An indexing mechanism according to any preceding claim, wherein the driving element (70,670) engages with a slot (30,630) in the counting component (20,620), and wherein the slot (30,630) comprises a cam surface (32,632) for engagement with the driving element (70,670).

7. An indexing mechanism according to any preceding claim, further comprising a restoring element (52,72,668) providing a biasing force to urge the locking (46,646) and/or driving element (70,670) back to the initial position.

8. An indexing mechanism according to claim 7, wherein the restoring element (52,72,668) comprises a leaf spring which is part of an integrally formed component which also comprises the locking (46,646) and/or driving element (70,670).

9. An indexing mechanism according to any preceding claim, wherein the locking and driving elements (46,70) are provided on separate components (40,60).

10. An indexing mechanism according to any of claims 1 to 8, wherein the locking and driving elements (646,670) are provided on a common component (650).

11. A method of indexing a counter comprising, in sequence, the steps of:
A. engaging a locking element (46,246) with a counting component (20,620) to prevent movement of the counting component (20,620); and subsequently
B. moving a driving element (70,670) to apply a motive force the counting component (20,620); and
C. moving the locking element (46,646) out of engagement with the counting component (20,620); wherein
the locking (46,646) and driving (70,670) elements are moved in steps B and C by movement of a common element (16,166); wherein
the driving element (70,670) applies said motive force to the counting component (20,620) before the locking element (46,646) fully moves out of engagement with the counting component (20,620); and wherein
the driving element (70,670) drives movement of the counting component (20,620) once the locking element (46,646) is fully out of engagement with the counting component (20,620).

12. A method of indexing a counter according to claim 11, wherein the locking (46,646) and driving (70,670) elements are moved simultaneously in steps B and C.

13. A method of indexing a counter according to claim 11 or 12, wherein step B comprises storing energy to drive movement of the counting component (20,620).

14. A method of indexing a counter according to any of claims 11 to 13, wherein the counting component (20,620) is rotatable and wherein the driving element (70,670) drives rotation of the counting component (20,620) and holds the counting component (20,620) in a rotated position after driving rotation.

15. A method of indexing a dose counter according to any of claims11 to 14, comprising the further step of:
D. moving the locking element (46,646) back into engagement with the counting component (20,620), for example by a spring biasing force.

16. A method of indexing a counter according to any of claims 11 to 15, wherein movement of the locking (46,646) and/or driving (70,670) element comprises rotation around a pivot (42,62,626).

## Patentansprüche

1. Dosiszählmechanismus für einen Zähler (10), bestehend aus einem Zählwerkbauteil (20, 620), einem Verriegelungselement (46, 646) zur Verhinderung der Bewegung der Zählwerkbauteil und einem separaten Antriebselement (70, 670) zum Antrieb der Bewegung der Zählkomponente, wobei die Verriegelungs- und der Antriebselemente eine Ausgangsposition aufweisen, in der das Verriegelungselement (46, 646) mit dem Zählwerkbauteil (20, 620) eingerastet ist,
**dadurch gekennzeichnet, dass** die Sperr- und Antriebselemente durch die Bewegung eines gemeinsamen Elements (16, 666) betätigt werden, um das Verriegelungselement (46, 646) aus dem Eingriff mit dem Zählwerkbauteil (20, 620) zu bewegen, und zum Bewegen des Antriebselements (70, 670), wobei das Antriebselement (70, 670) eine Antriebskraft auf das Zählwerkbauteil (20, 620) ausübt, bevor das Verriegelungselement (46, 646) sich vollständig von dem Zählwerkbauteil (20, 620) löst.

2. Dosiszählmechanismus nach Anspruch 1, wobei
der Eingriff des Verriegelungselements (46, 646) mit dem Zählwerkbauteil (20, 620) eine Bewegung des Zählwerkbauteils (20, 620) verhindert.

3. Dosiszählmechanismus nach Anspruch 1 oder 2, wobei
das Antriebselement (70, 670) an einem elastischen Arm (68, 368, 468, 668) angebracht ist, der sich vorzugsweise verformt, wenn das Antriebselement (70, 670) in das Zählelement (20, 620) zur Speicherung von potenzieller Energie im elastischen Arm (68, 368, 468, 668) eingreift.

4. Dosiszählmechanismus nach Anspruch 3, wobei
der elastische Arm (68, 368, 468, 668) einen klappbaren oder gebogenen Abschnitt (74, 374, 474) umfasst.

5. Dosiszählmechanismus nach Anspruch 4, wobei
der elastische Arm (68, 368, 468, 668) ein Widerlager (378, 478) umfasst, um einer Verformung des gelenkigen oder gebogenen Teils (374, 474) in eine erste Richtung entgegenzuwirken.

6. Dosiszählmechanismus nach einem der vorhergehenden Ansprüche, wobei
das Antriebselement (70, 670) in einen Schlitz (30, 630) des Zählwerkbauteils (20, 620) eingreift, und wobei der Schlitz (30, 630) eine Nockenfläche umfasst (32, 632) für den Eingriff mit dem Antriebselement (70, 670).

7. Dosiszählmechanismus nach einem der vorhergehenden Ansprüche, ferner mit einem Rückstellelement (52, 72, 668), das eine Vorspannkraft erzeugt, um die das Verriegelungs-(46, 646) und/oder Antriebselement (70, 670) zurück in die Ausgangsstellung zu bringen.

8. Dosiszählmechanismus nach Anspruch 7, wobei
die Rückstellungselement (52, 72, 668) aus einer Blattfeder besteht, die Teil eines integralen Formteils ist, das auch die Verriegelung (46, 646) und/oder das Antriebselement (70, 670) umfasst.

9. Dosiszählmechanismus nach einem der vorhergehenden Ansprüche, wobei die Verriegelungs- und Antriebselemente (46, 70) an separaten Bauteilen (40, 60) vorgesehen sind.

10. Dosiszählmechanismus nach einem der Ansprüche 1 bis 8, wobei
die Verriegelungs- und Antriebselemente (646, 670) an einem gemeinsamen Bauteil vorgesehen sind (650).

11. Verfahren zum Indizieren eines Dosiszählers, das nacheinander die folgenden Schritte umfasst:
A. Ineinandergreifen eines Verriegelungselements (46, 246) mit einer Zählwerkbauteil (20, 620), um eine Bewegung der Zählwerkbauteil (20, 620) zu verhindern; und nachfolgend
B. ein Antriebselement (70, 670) zu bewegen, um eine Bewegungskraft auf das Zählwerkbauteil (20.620) aufzubringen; und
C. das Verriegelungselement (46, 646) aus dem Eingriff mit dem Zählwerkbauteil (20, 620) zu bringen; wobei
die Verriegelungs- (46, 646) und Antriebselemente (70, 670) in den Schritten B und C bewegt werden durch Bewegung eines gemeinsamen Elements (16, 166); wobei das Antriebselement (70, 670) die Bewegungskraft auf die Zählung ausübt Komponente (20,620), bevor sich das Verriegelungselement (46,646) vollständig aus dem Eingriff mit der Zählwerkbauteil (20, 620); und wobei das Antriebselement (70, 670) die Bewegung des Zählwerkbauteils (20,620) antreibt, sobald das Verriegelungselement (46,646) vollständig aus dem Eingriff mit dem Zählwerkbauteil (20, 620) ist.

12. Verfahren zum Indizieren eines Dosiszählers nach Anspruch 11, wobei
die Verriegelungs- (46, 646) und Antriebselemente (70.670) gleichzeitig in den Schritten B und C bewegt werden.

13. Verfahren zum Indizieren eines Dosiszählers nach Anspruch 11 oder 12, wobei Schritt B die Speicherung von Energie umfasst, um das Zählwerkbauteil (20.620) anzutreiben.

14. Verfahren zum Indizieren eines Dosiszählers nach einem der Ansprüche 11 bis 13, wobei das Zählwerkbauteil (20, 620) drehbar ist, und wobei das Antriebselement (70, 670) die Drehung des Zählwerkbauteils (20, 620) antreibt und das Zählwerkbauteil (20, 620) nach dem Antrieb in einer gedrehten Position hält.

15. Verfahren zur Indexierung eines Dosiszählers nach einem der Ansprüche 11 bis 14, umfassend den weiteren Schritt:
D. Zurückbewegen des Verriegelungselements (46, 646) aus dem Zählwerkbauteil (20, 620), zum Beispiel durch eine Federvorspannung.

16. Verfahren zum Indizieren eines Dosiszählers nach einem der Ansprüche 11 bis 15, wobei die Bewegung des Verriegelungs- (46, 646) und/oder Antriebselements (70, 670) die Drehung um einen Drehpunkt (42,62,626) umfasst.

## Revendications

1. Un mécanisme d'indexation pour un compteur (10), comprenant un composant de comptage (20, 620), un élément de verrouillage (46, 646) pour résister au mouvement du composant de comptage et un élément d'entraînement distinct (70, 670) pour entraîner le mouvement du composant de comptage, les éléments de verrouillage et d'entraînement ayant une position initiale dans laquelle l'élément de verrouillage (46, 646) est en engagement avec le composant de comptage (20, 620), **caractérisé en ce que** les éléments de verrouillage et d'entraînement sont actionnés par le mouvement d'un élément commun (16, 666) pour déplacer l'élément de verrouillage (46, 646) hors d'engagement avec le composant de comptage (20, 620) et pour déplacer l'élément d'entraînement (70, 670), l'élément d'entraînement (70, 670) appliquant une force motrice au composant de comptage (20, 620) avant que l'élément de verrouillage (46, 646) se désengage complètement du composant de comptage (20, 620).

2. Un mécanisme d'indexation selon la revendication 1, dans lequel l'engagement de l'élément de verrouillage (46, 646) avec le composant de comptage (20, 620) empêche le mouvement du composant de comptage (20, 620).

3. Un mécanisme d'indexation selon la revendication 1 ou la revendication 2, dans lequel l'élément d'entraînement (70, 670) est prévu sur un bras élastique (68, 368, 468, 668) qui est de préférence déformé lors de la venue en engagement de l'élément d'entraînement (70, 670) avec l'élément de comptage (20, 620) afin de stocker de l'énergie potentielle dans le bras élastique (68, 368, 468, 668).

4. Un mécanisme d'indexation selon la revendication 3, dans lequel le bras élastique (68, 368, 468, 668) comprend une section articulée ou courbée (74, 374, 474).

5. Un mécanisme d'indexation selon la revendication 4, dans lequel le bras élastique (68, 368, 468, 668) comprend un élément de butée (378, 478) pour résister à la déformation de la section articulée ou courbée (374, 474) dans une première direction.

6. Un mécanisme d'indexation selon l'une quelconque des revendications précédentes, dans lequel l'élément d'entraînement (70, 670) est en engagement dans une fente (30, 630) dans le composant de comptage (20, 620), et dans lequel la fente (30, 630) comprend une surface de came (32, 632) pour un engagement avec l'élément moteur (70, 670).

7. Un mécanisme d'indexation selon l'une quelconque des revendications précédentes, comprenant en outre un élément de rappel (52, 72, 668) fournissant une force de sollicitation pour rappeler l'élément de verrouillage (46, 646) et/ou l'élément d'entraînement (70, 670) vers la position initiale.

8. Un mécanisme d'indexation selon la revendication 7, dans lequel l'élément de rappel (52, 72, 668) comprend un ressort à lame qui fait partie d'un composant formé d'un seul tenant qui comprend également l'élément de verrouillage (46, 646) et/ou l'élément d'entraînement (70, 670).

9. Un mécanisme d'indexation selon l'une quelconque des revendications précédentes, dans lequel les éléments de verrouillage et d'entraînement (46, 70) sont prévus sur des composants séparés (40, 60).

10. Un mécanisme d'indexation selon l'une quelconque des revendications 1 à 8, dans lequel les éléments de verrouillage et d'entraînement (646, 670) sont prévus sur un composant commun (650).

11. Un procédé d'indexation d'un compteur comprenant, en séquence, les étapes consistant à :
A. mettre en engagement un élément de verrouillage (46, 246) avec un composant de comptage (20, 620) pour empêcher le mouvement du composant de comptage (20, 620) ; et, ensuite
B. déplacer un élément d'entraînement (70, 670) pour appliquer une force motrice au composant de comptage (20, 620) ; et
C. déplacer l'élément de verrouillage (46, 646) hors d'engagement avec le composant de comptage (20, 620) ;
les éléments de verrouillage (46, 646) et d'entraînement (70, 670) étant déplacés dans les étapes B et C par le mouvement d'un élément commun (16, 166) ;
l'élément d'entraînement (70, 670) appliquant ladite force motrice au composant de comptage (20, 620) avant que l'élément de verrouillage (46, 646) ne se désengage complètement du composant de comptage (20, 620) ; et
l'élément d'entraînement (70, 670) entraîne le mouvement du composant de comptage (20, 620) une fois que l'élément de verrouillage (46, 646) soit complètement désengagé d'avec le composant de comptage (20, 620).

12. Un procédé d'indexation d'un compteur selon la revendication 11, dans lequel les éléments de verrouillage (46, 646) et d'entraînement (70, 670) sont déplacés simultanément dans les étapes B et C.

13. Un procédé d'indexation d'un compteur selon la revendication 11 ou 12, dans lequel l'étape B comprend le fait de stocker de l'énergie pour entraîner le mouvement du composant de comptage (20, 620).

14. Un procédé d'indexation d'un compteur selon l'une quelconque des revendications 11 à 13, dans lequel le composant de comptage (20, 620) est rotatif et dans lequel l'élément d'entraînement (70, 670) entraîne la rotation du composant de comptage (20, 620) et maintient le composant de comptage (20, 620) dans une position de rotation après avoir entraîné la rotation.

15. Un procédé d'indexation d'un compteur de doses selon l'une quelconque des revendications 11 à 14, comprenant l'étape supplémentaire consistant à :
D. remettre en engagement l'élément de verrouillage (46, 646) avec le composant de comptage (20, 620), par exemple par une force de sollicitation d'un ressort.

16. Un procédé d'indexation d'un compteur selon l'une quelconque des revendications 11 à 15, dans lequel le déplacement de l'élément de verrouillage (46, 646) et/ou d'entraînement (70, 670) comprend une rotation autour d'un pivot (42, 62, 626).
